# EUROPEAN PATENT APPLICATION

(11) **EP 0 923 907 A1**
(43) Date of publication of application: **23.06.1999**
(21) Application number: 98310161.9
(22) Date of filing: 11.12.1998
(51) Int. Cl.: A61B 17/39

(54) **An electrosurgical instrument**

(30) Priority: 19.12.1997 GB 9726952; 07.07.1998 GB 9814727
(71) Applicant: GYRUS MEDICAL LIMITED, Cardiff CF3 0LT (GB)
(72) Inventor: Goble, Nigel Mark, Castelton, Cardiff CF3 8SB (GB); Goble, Colin Charles Owen, Penarth, South Glamorgan CF64 1AT (GB); Syrop, Alain Nigel, Llandaff, Cardiff CF5 2LD (GB)
(74) Representative: Pratt, David Martin

(57) **Abstract**

An electrosurgical instrument for the treatment of tissue comprises an instrument shaft and an electrode assembly at the distal end of the shaft. The electrode assembly comprises an active electrode (11, 21) having a tissue treatment portion, a return electrode (12, 22) and an insulating member (14, 24) electrically insulating the active electrode and the return electrode. The active electrode (11, 21 )is movable cyclically relative to the return electrode (12, 22) so as to move the active electrode cyclically into, and out of, a position in which arcing occurs between the active and return electrodes. The active electrode (11, 21) may be rotatable or reciprocable relative to the return electrode (12, 22).

## Description

This invention relates to an electrosurgical instrument for the treatment of tissue in an open-air or gas-filled environment, to electrosurgical apparatus including such an instrument, and to an electrode unit for use in such an instrument.

Endoscopic electrosurgery is useful for treating tissue in cavities of the body, and is normally performed in the presence of a distension medium. When the distension medium is a liquid, this is commonly referred to as underwater electrosurgery, this term denoting electrosurgery in which living tissue is treated using an electrosurgical instrument with a treatment electrode or electrodes immersed in liquid at the operation site. A gaseous medium is commonly employed when endoscopic surgery is performed in a distensible body cavity of larger potential volume in which a liquid medium would be unsuitable, as is often the case in laparoscopic or gastroenterological surgery.

Electrosurgery is usually carried out using either a monopolar instrument or a bipolar instrument. With monopolar electrosurgery, an active electrode is used in the operating region, and a conductive return plate is secured to the patent's skin. With this arrangement, current passes from the active electrode through the patient's tissues to the external return plate. Since the patient represents a significant portion of the circuit, input power levels have to be high (typically 150 to 250 watts), to compensate for the resistive current limiting of the patient's tissues and, in the case of underwater electrosurgery, power losses due to the fluid medium which is rendered partially conductive by the presence of blood or other body fluids. Using high power with a monopolar arrangement is also hazardous, due to the tissue heating that occurs at the return plate, which can cause severe skin burns. There is also the risk of capacitive coupling between the instrument and patient tissues at the entry point into the body cavity.

With bipolar electrosurgery, a pair of electrodes (an active electrode and a return electrode) are used together at the tissue application site. This arrangement has advantages from the safety standpoint, due to the relative proximity of the two electrodes so that radio frequency currents are limited to the region between the electrodes. However, the depth of effect is directly related to the distance between the two electrodes, and, in applications requiring very small electrodes, the inter-electrode spacing becomes very small, thereby limiting tissue effect and output power. Spacing the electrodes further apart would often obscure vision of the application site, and would require a modification in surgical technique to ensure correct contact of both electrodes with tissue.

The electrical junction between the return electrode and tissue can be supported by wetting of the tissue by a conductive solution such as normal saline. This ensures that the surgical effect is limited to the needle or active electrode, with the electric circuit between the two electrodes being competed by the tissue. One of the obvious limitations with the design is that the needle must be completely buried in the tissue to enable the return electrode to complete the circuit. Another problem is one of the orientation; even a relatively small change in application angle from the ideal perpendicular contact with respect to the tissue surface, will change the contact area ratio, so that a surgical effect can occur in the tissue in contact with the return electrode.

Cavity distension provides space for gaining access to the operation site, to improve visualisation, and to allow for manipulation of instruments. Carbon dioxide is the preferred gaseous distension medium, primarily because of its non-toxic nature and high water solubility.

The present invention provides an electrosurgical instrument for the treatment of tissue, the instrument comprising an instrument shaft and an electrode assembly at the distal end of the shaft, the electrode assembly comprising an active electrode having a tissue treatment portion, a return electrode, and insulating means for electrically insulating the active electrode from the return electrode, wherein the active electrode is movable cyclically relative to the return electrode so as to move the active electrode cyclically into, and out of, a position in which arcing occurs between the active and return electrodes.

Advantageously, the insulation means is an insulation member positioned between and electrically insulating the active electrode and the return electrode. Preferably, the active electrode is fixed to the insulating member for movement therewith, and the insulating member is movable cyclically relative to the return electrode, thereby causing the active electrode to move cyclically into, and out of, said position.

The instrument may further comprise a motor for cyclically moving the insulating member. Preferably, the motor is effective to rotate the insulating member continuously in the same direction. Alternatively, the motor is effective to rotate the insulating member alternately in the clockwise and anti-clockwise directions.

In a preferred embodiment, the return electrode is generally cup-shaped, and is formed with a cut-out at its distal end portion, and the insulating member is generally cylindrical and is formed with a cut-out which houses the active electrode. Advantageously, the insulating member is formed with a channel for delivering cooling fluid to an operation site adjacent to the active electrode, and with an axially-extending channel which is connectable to a suction pump.

The distal end of the suction channel may be adjacent to the active electrode, and the suction channel may be positioned at the leading edge of the active electrode.

In a preferred embodiment, the active electrode is reciprocable relative to the return electrode. In this case, the return electrode may be a generally tubular member, the active electrode may be a generally U-shaped member having a base and a pair of generally parallel legs, the base being positioned within a recess formed within the distal end portion of the return electrode, and the each of the legs of the U-shaped member may be mounted within a respective insulating sleeve. Conveniently, the insulating sleeves are located on the outside of the return electrode, and preferably the insulating sleeves are fixed to the return electrode.

Preferably, the legs of the U-shaped member are axially reciprocable relative to their insulating sleeves.

Advantageously, the active electrode is reciprocable within the return electrode so as to move the active electrode cyclically into, and out of two positions in which arcing occurs between the active and return electrodes, the two positions being defined by the end positions of the reciprocal movement of the active electrode.

A motor may be provided for reciprocating the active electrode. Conveniently, the motor is an electric motor, or a device for converting energy from a pressurised fluid into mechanical motion.

This form of instrument may further comprise a suction pump connected to the proximal end of the tubular member constituting the return electrode, and delivery means for delivering cooling fluid to an operation site adjacent to the active electrode. Preferably, a tube fixed to the outside of the tubular return electrode constitutes the delivery means, and the delivery means further comprises aligned apertures formed in the tube and in the tubular return electrode, the aligned apertures being positioned to direct cooling fluid into the recess formed within the distal end portion of the return electrode.

Preferably, the arrangement is such that, when the active electrode is out of said position(s) in which shorting occurs, the active electrode is spaced from the return electrode by a distance of substantially 1mm.

The invention will now be described in greater detail, by way of example, with reference to the drawings, in which:-
Figure 1 is a diagram showing an electrosurgical apparatus constructed in accordance with the invention;
Figure 2 is a front elevation of the distal end portion of a first form of electrosurgical instrument which can form part of the apparatus of Figure 1;
Figure 3 is a front elevation of a ceramic insulator forming part of the instrument of Figure 2;
Figure 4 is a plan view of the ceramic insulator;
Figure 5 is a transverse cross-section taken through the ceramic insulator;
Figure 6 is a front elevation of the distal end portion of a second form of electrosurgical instrument which can form part of the apparatus of Figure 1;
Figure 7 is a part-sectional side view of the instrument of Figure 6; and
Figure 8 is a perspective view of the distal end of the second form of electrosurgical instrument.

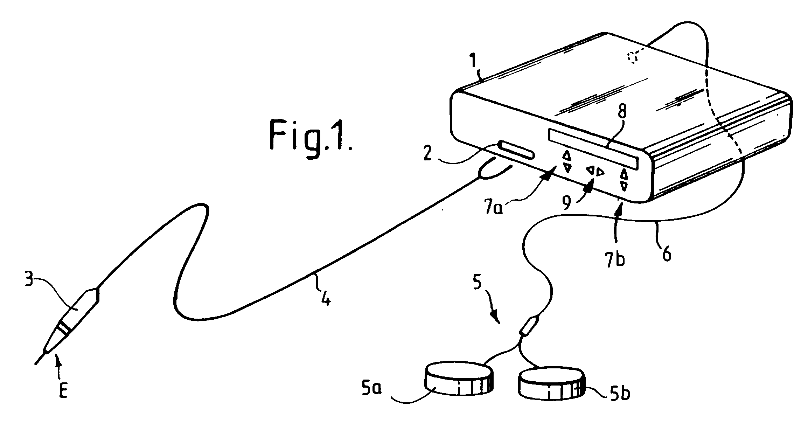

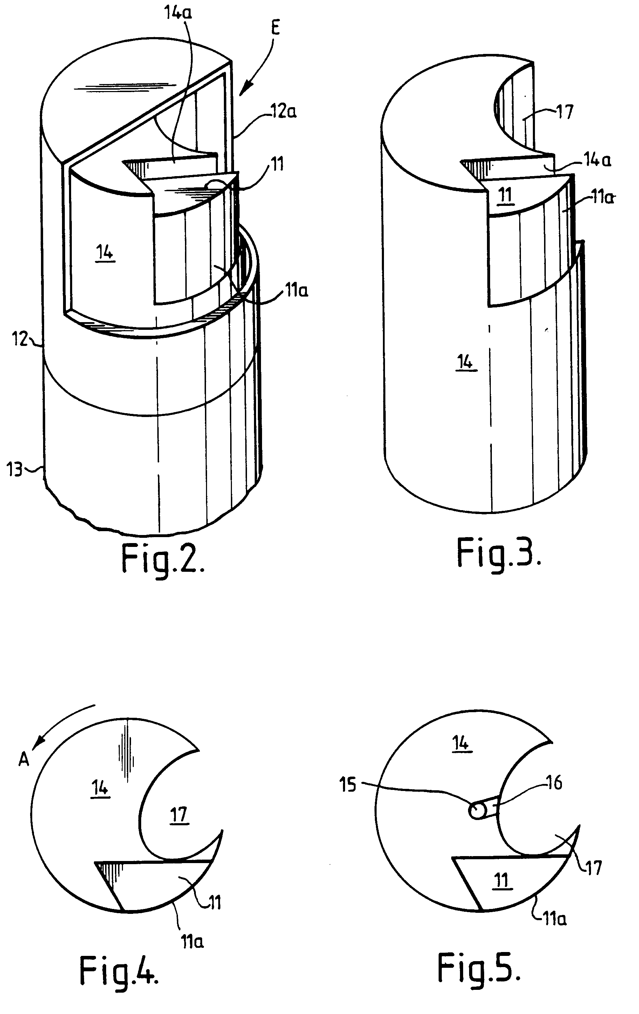

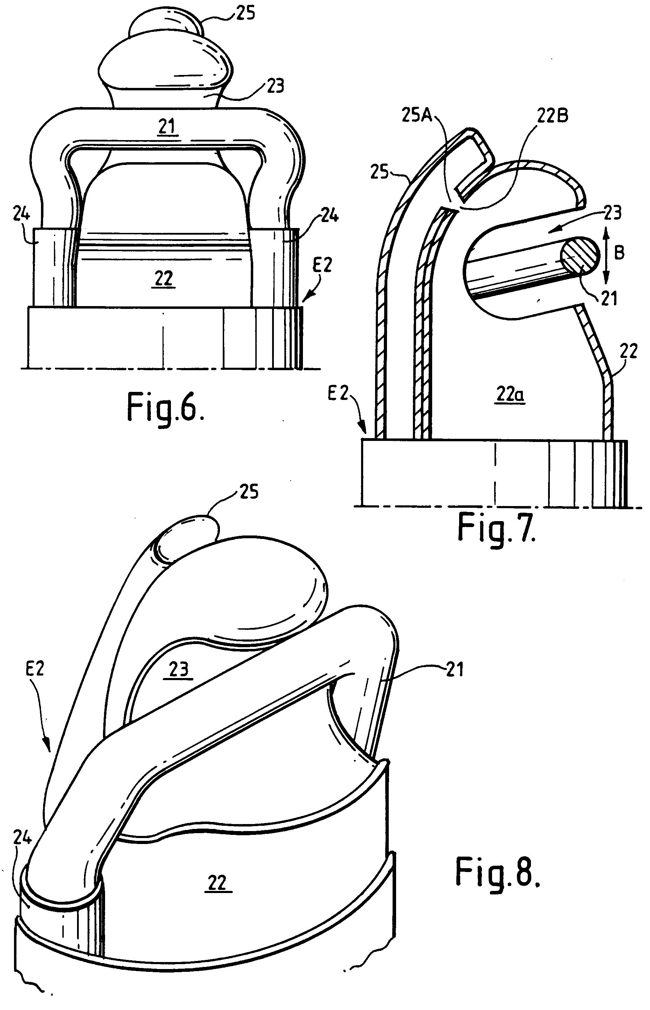

Referring to the drawings, Figure 1 shows an electrosurgical apparatus including a generator 1 having an output socket 2 providing a radio frequency (RF) output for an instrument in the form of a handpiece 3 via a connection cord 4. Activation of the generator 1 may be performed from the handpiece 3 via a control connection in the cord 4, or by means of a footswitch unit 5, as shown, connected separately to the rear of the generator 1 by a footswitch connection cord 6. In the illustrated embodiment, the footswitch unit 5 has two footswitches 5a and 5b for selecting a desiccation mode and a vaporisation mode of the generator 1 respectively. The generator front panel has push buttons 7a and 7b for respectively setting desiccation and vaporisation power levels, which are indicated in a display 8. Push buttons 9a are provided as an alternative means for selection between the desiccation and vaporisation modes. The handpiece 3 mounts a detachable electrode unit E1 which is described below with reference to Figures 2 to 5, or a detachable electrode unit E2 which is described below with reference to Figures 6 to 8.

Figure 2 shows the distal end portion/electrode assembly of the electrode unit E1. The electrode assembly comprises a central tissue treatment (active) electrode 11 made of stainless steel, and a cup-shaped return electrode 12 also made of stainless steel. The return electrode 12 is fixed to the distal end of an insulated member 13 constituting the shaft of the instrument. The return electrode 12 is formed with a cut-out 12a at the distal end thereof The active electrode 11 is fixed within a complementary cut-out 14a formed in a ceramic insulator 14 rotatably mounted within the return electrode 12, so that the distal end of the ceramic insulator (including the active electrode) is positioned within the region of the cut-out 12a. The active electrode 11 has a curved outer surface 11a which is contiguous with the generally cylindrical outer surface of the ceramic insulator 14. The ceramic insulator 14 is sized so that there is a gap of about 1mm between its cylindrical outer surface and the cylindrical inner surface of the cup-shaped return electrode 12.

The ceramic insulator 14 is formed with an axial delivery passage 15 which terminates in the region of the active electrode 11. A radial delivery passage 16 connects the axial passage 15 to an axially-extending channel 17 formed in the ceramic insulator 14. The passages 15 and 16 and the distal end of the channel 17 define a fluid flow path for a cooling fluid to be delivered to an operation site adjacent to the active electrode 11 via the interior of the instrument body. The channel 17 is connected to a vacuum pump (not shown), and so constitutes a suction channel for removing cooling fluid, gas and tissue debris from the operation site in the manner to be described below.

The ceramic insulator 14 is rotatably driven by a motor (not shown) housed within the handpiece 3. The electrosurgical instrument can be used for tissue removal by vaporisation or for desiccation. In either case, the instrument is used in an open-air or gas-filled environment. In the latter case, the gas is preferably an inert gas such as argon. When the ceramic insulator 14 is stationary, with the active electrode positioned within the cut out 12a (as shown in Figure 2), the generator 1 can be activated by the footswitch 5a or the button 7a to set a suitable desiccation power level. Tissue can then be desiccated by moving the electrode assembly over the tissue at an operation site with both active and return electrodes 11 and 12 in contact with the tissue.

When the instrument is used in the vaporisation mode, the generator 1 is activated by the footswitch 5b or the button 7b to set a suitable vaporisation power level. At the same time, the motor is activated to rotate the ceramic insulator 14 and the active electrode 11. Tissue can then be vaporised at an operation site with both the active and the return electrodes 11 and 12 in contact with the tissue. As the assembly rotates, the active electrode 11 moves from the position shown in Figure 2, in which it is spaced from the return electrode 12 by 1 mm, rotating in the direction of the arrow A (see Figure 4), so as to lie in close proximity (that is to say much less than 1 mm) with the return electrode, thereby shorting the active and return electrodes. Although the shorting is not particularly beneficial, the increasing field intensity as the active electrode 11 approaches the return electrode 12 ensures that any tissue removed by the leading edge of the active electrode is subjected to vigorous arcing, and hence vaporisation, before the electrical short occurs. The active electrode 11 is, therefore, moved cyclically relative to the return electrode 12 so as to move the active electrode cyclically into, and out of, a position in which arcing occurs between the active and return electrodes. The suction channel 17 is situated at the leading edge of the active electrode 11 as it approaches the return electrode 12, so that the suction is effective at removing vaporised tissue from the operation site, together with cooling fluid and particulate material. Moreover, as the arc intensity increases as the active electrode 11 approaches the return electrode 12, the effective size of the suction channel 17 is reduced, thereby increasing the suction velocity at the leading edge of the active electrode, and so increasing the effectiveness of the suction. Thus, as the ceramic insulator 14 rotates, the tissue engaged by the active electrode 11 is subjected to varying degrees of both electrical and mechanical stress. The increasing electrical and mechanical intensity as the active electrode 11 approaches the return electrode 12 reduces the sensitivity of the electrode assembly to different tissue types and impedances.

It will be appreciated that the arcing and the shorting that occurs at the electrode tip produces a significant build-up of heat. The delivery of cooling fluid to the operation site via the channels 15 and 16, and the removal of this fluid via the channel 17, provides adequate cooling of the tip to limit this build-up of heat. The electrical behaviour of the cooling fluid is virtually irrelevant, because it is present in only small amounts, forming in effect a thin film in the region of the active electrode 11. Consequently, either saline or glycine can be used as the cooling fluid.

Applying suction to the tip directly effects tissue engagement. As there is no danger of quenching the arcing at the tip, it is possible, and even desirable, to use as high a level of suction a possible. The use of high suction levels, however, can prevent disengagement of the active electrode 11 from tissue. It is, therefore, advisable to operate the vacuum pump in conjunction with the RF generator 1, thereby ensuring that the tip of the instrument can be disengaged from the tissue as soon as the RF power is turned off

The intermittent shorting of the two electrodes 11 and 12 can place a heavy demand on the generator 1, and so this must be carefully controlled. It is also necessary to current limit the generator 1 to prevent both excessive current and to reduce erosion of the electrode surfaces.

In a modified form of instrument (not shown), the ceramic insulator 14 is formed with a second fluid supply channel which terminates at the proximal end of the active electrode 11, and leads into the suction channel 17. Fluid (such as saline) is supplied to this additional channel at a faster rate than to the channel 15. The fluid passing through the additional channel act as a condensing medium for vaporised tissue, and to assist with the removal of smoke and particulate materials.

In another modified form of instrument (not shown), the ceramic insulator 14 (and hence the active electrode 11) is rotatably driven back and forth through an angle of less than 360°. The angle through which the active electrode 11 is driven back and forth depends, inter alia, upon the size of the active electrode and the size of the gap between the cylindrical outer surface of the insulator 14 and the cylindrical inner surface of the return electrode 12. Typically, the angle is less than 90°, for example 25°. Here again, therefore, the active electrode is moved cyclically relative to the return electrode so as to move the active electrode cyclically into, and out of, a position in which arcing occurs, between the active and return electrodes.

Figures 6 to 8 show the distal end portion/electrode assembly of the electrode unit E2. The electrode assembly comprises a generally U-shaped tissue treatment (active) electrode 21 made of stainless steel, and a hollow return electrode 22 also made of stainless steel. The distal end portion of the return electrode 22 is formed with a U-shaped aperture 23 in which the active electrode 21 is reciprocal as indicated by the double-headed arrow B. The proximal end portions of the two legs of the U-shaped active electrode 21 are supported in a pair of insulating sleeves 24 fixedly mounted at opposite sides on the exterior of the return electrode 22. The interior 22A of the return electrode 22 constitutes a suction channel for removing cooling fluid, gas and tissue debris from the operation site in the manner to be described below. A tube 25, which has a circular cross-section, extends along the exterior of the return electrode 22 on the side opposite the U-shaped aperture 23. Aligned apertures 25A and 22B in the tube 25 and the return electrode 22 constitutes a passage for the delivery of cooling fluid from the tube to the interior of the return electrode, and hence to the active electrode 21.

The active electrode 21 is reciprocally driven by an actuator (not shown), which can be one of many devices capable of converting energy from a pressurised fluid or from electrical energy into mechanical motion. Advantageously, for higher speeds, an electrical motor with a gear box and a linkage is used to provide the reciprocating motion of the active electrode 21. The legs of the active electrode 21 are reciprocable, in use, within the isulating sleeves 24. The required amplitude of motion is governed by the size and shape of the aperture 23, whilst the frequency is determined by the need to give the surgeon a reasonable smooth operation. A minimum frequency of 1 Hz is required, whilst the maximum frequency is limited by frictional heating of the bearing and sealing surfaces. The preferred range of frequencies is from about 5 Hz to about 30 Hz. The active electrode 21 is, therefore, moved cyclically relative to the return electrode 22 so as to move the active electrode cyclically into, and out of, end positions in which arcing occurs between the active and return electrodes.

In use, cooling fluid (such as saline or glycine) is fed to an operation site adjacent to the active electrode 21 via the tube 25, the aligned apertures 25A and 22B and the U-shaped aperture 23. The proximal end of the hollow return electrode 22 is connected to a vacuum pump (not shown) thereby constituting a suction channel as mentioned above. The reciprocation of the active electrode 21 allows the gap between the active electrode and the return electrode 22 to be varied in such a way that arcing can be made to occur most of the time when the active electrode is engaging tissue. This is advantageous, particularly when the electrode unit E2 is used in the vaporisation mode. Thus, the length of time of high field intensity as the active electrode 21 approaches the return electrode 22 in this embodiment ensures that any tissue removed by the active electrode is subjected to prolonged vigourous arcing and hence vaporisation, before an electrical short occurs. As with the electrode unit E1, the suction channel 22A is effective to removed vaporised tissue from the operation site together with cooling fluid and particulate material.

This ability of the electrode unit E2 to remove cut strands of tissue or incompletely vaporised debris from the operated site, is most useful to the surgeon. This ability is enhanced by the use of a using high level of suction to aspirate the distal end of the unit. This is most advantageously achieved using a vacuum pump and a water (or debris) trap (commonly referred to as a suction canister). This maximises the removal of cooling fluid, debris etc., and minimises the risk of blockage. This action can be further enhanced by the use of an excess of cooling fluid to flush the aspiration channel. This excess supply of cooling fluid also enhances the removal of excess heat from the region of the operation site. Overall, a flow rate of cooling fluid of between 0.1 and 1 ml per second is appropriate, dependent upon the diameter of the aspiration channel.

Typically, the high levels of suction employed will cause soft tissue to become attached to the mouth of the aspiration channel, thereby preventing the surgeon from controlling the position of the distal end of the electrode unit E2. In order to prevent this, either a second (small) aperture is provided in the aspiration channel in a position which does not, in use, contact tissue. Alternatively, the vacuum pump is pulsed, for example by using a pinch valve, which can be turned on or off periodically, in the downstream pipework.

Although the use of an electrical motor for reciprocating the active electrode 21 is preferred for high speed operation, it is also possible to use the flow of cooling fluid or the aspiration flow as the medium of energy transfer to the active electrode 21. This has the advantage of reducing the number of connections between the handpiece of the electrosurgical instrument and the associated generator. One way of achieving this would be use of a turbine, a gear box and a linkage. Alternatively and preferably, pressure pulsations in the cooling fluid flow could be used to actuate the active electrode via a cylinder, a resilient pouch, a diaphragm or similar means to convert flow to mechanical movement.

In a modified form (not shown) of the reciprocating active electrode embodiment of Figures 6 to 8, the active electrode is driven to simulate the movement of a windscreen wiper blade. Thus, the active electrode is formed as an arm extending generally at right-angles to a rod which is rotatably driven back and forth, through a small angle, about its own axis. The active electrode is positioned within a sector-shaped recess formed in the return electrode. As with the embodiment of Figures 6 to to 8, the active electrode is moved cyclically into, and out of, end positions in which arcing occurs between the active and return electrodes.

The electrosurgical instruments described above are particularly useful for laparoscopic and ENT surgery.

## Claims

1. An electrosurgical instrument for the treatment of tissue, the instrument comprising an instrument shaft and an electrode assembly at the distal end of the shaft, the electrode assembly comprising an active electrode having a tissue treatment portion, a return electrode, and insulating means for electrically insulating the active electrode from the return electrode, wherein the active electrode is movable cyclically relative to the return electrode so as to move the active electrode cyclically into, and out of, a position in which arcing occurs between the active and return electrodes.

2. An instrument as claimed in claim 1, wherein the insulation means is an insulation member positioned between and electrically insulating the active electrode and the return electrode.

3. An instrument as claimed in claim 2, wherein the active electrode is fixed to the insulating member for movement therewith, and the insulating member is movable cyclically relative to the return electrode, thereby causing the active electrode to move cyclically into, and out of, said position.

4. An instrument as claimed in claim 2 or claim 3, further comprising a motor for cyclically moving the insulating member.

5. An instrument as claimed in claim 4, wherein the motor is mounted within the instrument shaft and is effective to rotate the insulating member.

6. An instrument as claimed in claim 5, wherein the motor is effective to rotate the insulating member continuously in the same direction.

7. An instrument as claimed in claim 5, wherein the motor is effective to rotate the insulating member alternately in the clockwise and anti-clockwise directions.

8. An instrument a claimed in any one of claims 2 to 7, wherein the return electrode is generally cup-shaped, and is formed with a cut-out at its distal end portion.

9. An instrument as claimed in claim 8, wherein the insulating member is generally cylindrical, and is formed with a cut-out which houses the active electrode.

10. An instrument as claimed in claim 9, wherein the insulating member is formed with a channel for delivering cooling fluid to an operation site adjacent to the active electrode.

11. An instrument as claimed in claim 10, wherein the insulating member is formed with an axially-extending channel which is connectable to a suction pump.

12. An instrument as claimed in claim 11, wherein the distal end of the suction channel lies adjacent to the active electrode.

13. An instrument as claimed in claim 12 when appendant to claim 8, wherein the suction channel is positioned at the leading edge of the active electrode.

14. An instrument as claimed in claim 1, wherein the active electrode is reciprocable relative to the return electrode.

15. An instrument as claimed in claim 14, wherein the return electrode is a generally tubular member.

16. An instrument as claimed in claim 15, wherein the active electrode is a generally U-shaped member having a base and a pair of generally parallel legs, the base being positioned within a recess formed within the distal end portion of the return electrode.

17. An instrument as claimed in claim 16, wherein each of the legs of the U-shaped member is mounted within a respective insulating sleeve.

18. An instrument as claimed in claim 17, wherein the insulating sleeves are located on the outside of the return electrode.

19. An instrument as claimed in claim 18, wherein the insulating sleeves are fixed to the return electrode.

20. An instrument as claimed in claim 19, wherein the legs of the U-shaped member are axially reciprocable relative to their insulating sleeves.

21. An instrument as claimed in any one of claims 14 to 20, wherein the active electrode is reciprocable within the return electrode so as to move the active electrode cyclically into, and out of, two positions in which arcing occurs between the active and return electrodes, the two positions being defined by the end positions of the reciprocal movement of the active electrode.

22. An instrument as claimed in any one of claims 14 to 21, further comprising a motor for reciprocating the active electrode.

23. An instrument as claimed in claim 22, wherein the motor is an electric motor.

24. An instrument as claimed in claim 22, wherein the motor is a device for converting energy from a pressurised fluid into mechanical motion.

25. An instrument as claimed in any one of claims 15 to 24, further comprising a suction pump connected to the proximal end of the tubular member constituting the return electrode.

26. An instrument as claimed in any one of claims 14 to 25, further comprising delivery means for delivering cooling fluid to an operation site adjacent to the active electrode.

27. An instrument as claimed in claim 26, wherein a tube fixed to the outside of the tubular return electrode constitutes the delivery means.

28. An instrument as claimed in claim 27 when appendant to claim 16, wherein the delivery means further comprises aligned apertures formed in the tube and in the tubular return electrode, the aligned apertures being positioned to direct cooling fluid into the recess formed within the distal end portion of the return electrode.

29. An instrument as claimed in any one of claims 1 to 28, wherein the arrangement is such that, when the active electrode is out of said position(s) in which shorting occurs, the active electrode is spaced from the return electrode by a distance of substantially 1mm.
